# NEUE EUROPÄISCHE PATENTSCHRIFT

(11) **EP 0 420 043 B2**
(45) Veröffentlichungstag und Bekanntmachung des Hinweises auf die Entscheidung über den Einspruch: **22.04.1998**
(45) Hinweis auf die Patenterteilung: 23.03.1994
(21) Anmeldenummer: 90118103.2
(22) Anmeldetag: 20.09.1990
(51) Int. Cl.: C12P 21/08, C07K 16/26, G01N 33/74

(54) **Antikörper gegen hochkonservierte Aminosäuresequenzen von immunogenen Substanzen, Verfahren zur Herstellung dieser Antikörper, sowie deren Verwendung in Immunoassays**
Antibodies against highly conservated amino acid sequences of immunogenic substances, process of preparation of these antibodies and their use as immunoessays
Anticorps contre des séquences très conservées d'amino-acides de substances immunogènes, procédé de préparation de ces anticorps et leur utilisation dans des immunoessais

(30) Priorität: 23.09.1989 DE 3931787; 30.05.1990 DE 4017344
(43) Veröffentlichungstag der Anmeldung: 03.04.1991
(73) Patentinhaber: HOECHST AKTIENGESELLSCHAFT, 65926 Frankfurt am Main (DE)
(72) Erfinder: Müllner, Stefan, Dr., D-6230 Frankfurt 80 (DE)

(56) Entgegenhaltungen:
- EP-A- 0 314 338
- BIOLOGICAL ABSTRACTS, Band 86, Nr. 10, 1988, Philadelphia, PA (US); D. PLACHOV et al., Nr. 103111#
- CHEMICAL ABSTRACTS, Band 102, Nr. 21, 27 May 1985, Columbus, OH (US); A. MARKS et al., Seite 90, Nr. 179524y#
- R Jemmerson et al. (1985), Proc natl Acad Sci, USA 82, 1508-1512
- G Walter (1986), J Immunol Methods 88, 149-161
- H L Niman (1984) Nature 307, 180-183
- T J Burke et al (1988) Mol Gen Genet 213, 435-443
- R D Vierstra et al (1987), Plant Gene Systems and their Biology, 251-259, Alan R Liss Inc
- U Bond et al (1986) Mol Cell Biol 6, 4602-4610
- P Karlson (1980) "Kurzes Lehrbuch der Biochemie", 11. Auflage
- E Harlow and D Lane (1988) "Antibodies, A Laboratory Manual", 72-76
- Tabelle 6-4 "Aminosäure-Squenzen von Cytochom C-Molekülen aus 38 Spezies", Literaturstellen sind in der Legende zur Tabelle 6-4 angegeben

## Beschreibung

Es ist bekannt, daß zum qualitativen und quantitativen Nachweis von immunogenen Substanzen, wie Antigenen, in immer größerem Maße immunometrische Methoden herangezogen werden. Diese Methoden beruhen auf der Bildung eines Komplexes der immunogenen Substanz mit einem oder mehreren Antikörpern, wobei einer der Bindungspartner zwecks Detektion markiert ist. Dadurch ist es möglich, festzustellen, ob und in welcher Menge sich ein Komplex aus der immunogenen Substanz und einem oder mehreren Antikörpern gebildet hat. Entscheidende Verbesserungen der immunometrischen Bestimmungsverfahren gelangen mit der Einführung monoklonaler Antikörper durch Milstein und Köhler, deren Anwendung in immunometrischen Assays in der deutschen Offenlegungsschrift 31 30 834 eingehend beschrieben ist. Auch immunometrische Verfahren zur Bestimmung von Insulinen bestimmter Spezies sind bereits beschrieben (J. Havrankova et al., Journal of Immunoassay, 5 (182), 131-144 (1984)). Diese Antikörper wurden durch Immunisierung mit den verschiedenen Insulinen als Immunogen erhalten. Die Verwendung von Antipeptidantikörpern, welche durch Immunisierung mit Bruchstücken des betreffenden Proteins (Antigens) erhalten wurden, zur Identifizierung von nativen Proteinen brachten weitere Verbesserungsmöglichkeiten hinsichtlich der Universalität immunometrischer Bestimmungen. Solche Antipeptidantikörper sind mittlerweile ein wichtiges Werkzeug bei der Identifikation von Peptiden und damit Gensequenzen.

Von besonderem Interesse sind diese Antikörper auch für die Bestimmung von Substanzen, gegen die auf herkömmliche Weise keine oder nur in beschränktem Umfang Antiseren generiert werden können, weil diese - als vollständige Proteine injiziert - entweder in den benötigten Konzentrationen schon zu hoch wirksam sind, z.B. Peptidhormone, Neuropeptide oder zu toxisch wirken, z.B.Diphtheria Toxin, Viren und andere Mikroorganismen. Bei der Entwicklung von "Synthetischen Impfstoffen" (J.G. Sutcliff et al., Science, Vol. 219, 660 (1983)) konnten solche Antipeptidantikörper ebenfalls erfolgreich eingesetzt werden. Für die Auswahl der Peptidsequenz gegen welche auf literaturbekannte Weise polyklonale oder monoklonale Antikörper gewonnen werden sollen, scheint es vorteilhaft zu sein, daß zumindest ein Teil dieser Sequenz an der Oberfläche des nativen Proteins lokalisiert - d.h. exponiert - ist und somit vermehrt geladene oder stark polare funktionelle Gruppen enthält. Es wird hierbei im Stand der Technik jedoch ausdrücklich darauf verwiesen, daß Peptidbruchstücke, die evolutionär hochkonservierte Aminosäuresequenzen darstellen, sehr schlechte Immunogene sind (s. G. Walter, J. Immunol. Med. 88, (1986), 149-161). Unter evolutionär hochkonservierten Aminosäuresequenzen werden hierbei solche Sequenzen eines gegebenen Proteins verstanden, die sich im Verlauf der Evolution nicht oder nur wenig verändert haben. So unterscheiden sich beispielsweise Pferde- und KaninchenCytochrom C in einigen Aminosäuresequenzen. Andere Sequenzen dieses Proteins aus den beiden Tierspezies sind hingegen identisch. Es wurde beobachtet, daß das Immunsystem der einen Tierspezies keine Antikörper gegen diese identischen Regionen des Cytochrom C der anderen Spezies bildet, da diese Sequenz ja auch in dem körpereigenen Cytochrom C vorkommt. Es gilt als Regel, daß die Immunantwort auf ein Antigen um so besser ist, je größer der evolutionäre Abstand zwischen immunisierendem Protein und dem entsprechenden körpereigenen Protein ist.

Der Erfindung lag nun die Aufgabe zugrunde, Antikörper zur Verfügung zu stellen, die in der Lage sind, sowohl mit einem nativen Protein Insulin, als auch mit Derivaten, Mutanten, denaturierten Produkten, Fragmenten oder (synthetischen) Vorstufen Immunkomplexe zu bilden.

Insbesondere bestand die Aufgabe darin, Antikörper zur Verfügung zu stellen, welche mit gentechnologisch hergestellten Insulin der verschiedensten Spezies sowie deren Derivaten, denaturierten Vorstufen und Fragmenten Immunkomplexe bilden.

Eine weitere Aufgabe bestand darin, ein immunometrisches Meßverfahren zu entwickeln, welches die Messung der Initialausbeute von gentechnologisch hergestellten Insulin, die in Mikroorganismen als schwerlösliche "Inclusion bodies" anfallen, erlaubt - was mit immunologischen Meßverfahren gemäß Stand der Technik bisher nicht möglich ist - und gleichzeitig in der Lage ist, die Proteinkonzentrationen in den einzelnen Aufarbeitungsstufen mit dem gleichen Meßverfahren zu messen. Unter Initialausbeute wird diejenige Ausbeute verstanden, welche direkt nach der Fermentation effektiv vorhanden ist. Sie ist nicht verfälscht durch Verluste in der Probenaufbereitung und durch Aufbereitungsstufen.

Überraschenderweise wurde nun gefunden, daß Antikörper, welche durch Immunisierung mit hochkonservierten Peptidbruchstücken des betreffenden nativen Proteins Insulin erhalten wurden, die obengenannte Aufgabe erfüllen.

Die Erfindung betrifft somit:

Antikörper, welche durch Immunisierung mit einem Peptidbruchstück, welches eine hochkonservierte Aminosäuresequenz eines nativen Proteins Insulin darstellt, erhalten werden.

Insbesondere betrifft die Erfindung solche Antikörper, die durch Immunisierung mit hochkonservierten Peptidbruchstücken des Insulins erhalten werden.

Weiterhin betrifft die Erfindung ein Verfahren zur Herstellung obengenannter Antikörper sowie die Verwendung derselben in immunometrischen Meßverfahren.

Im Vorstehenden wie im Folgenden werden unter hochkonservierten Aminosäuresequenzen solche Proteinfragmente eines gegebenen, in mehreren Spezies - wenn auch mehr oder weniger leicht modifiziert - vorkommenden Proteins verstanden, welche sich im Verlaufe der Evolution nicht oder gegebenenfalls nur unwesentlich verändert haben. Als Beispiel sei hier das Octapeptid (14-21) der Insulin-A-Kette genannt (Tyr-Glu-Leu-Glu-Asn-Tyr-Cys-Asn), welches in vielen bekannten Insulinen, wie Human-, Schweine-, Schaf-, Pferd-, Rinder-, Huhn-, Enten-, Truthahn-, Gans-, Aligator-, Klapperschlangen-, Colubrid-Snake-, Sei Whale-, Elefanten-, Ziegen-, Hunde-, Affen-, Sperm Whale-, Fin Whale-, Ratten-, Maus-, Hamster-, Kaninchen-Insulin unverändert vorkommt.

Unter einem nativen Protein wird ein natürlich vorkommendes Protein verstanden.

Unter Peptidbruchstücken, Peptidfragmenten, Proteinbruchstücken und Proteinfragmenten werden Teile eines betreffenden Peptids/Proteins verstanden, die natürlicher Bestandteil dieses Peptids/Proteins sind. Es handelt sich hierbei um zusammenhängende Aminosäuren (eine sogenannte Aminosäuresequenz), die einen Ausschnitt oder einen Anfang oder ein Ende des Peptids/Proteins darstellen.

Zur Herstellung der erfindungsgemäßen Antikörper gegen hochkonservierte Aminosäuresequenzen vom nativen Protein Insulin geht man am besten folgendermaßen vor:

### 1. Auswahl der betreffenden Aminosäuresequenz nach folgenden Kriterien:

a) Die betreffende Sequenz sollte bevorzugt exponiert sein, d.h., sie sollte sich an der Oberfläche des nativen Proteins befinden, welches bevorzugt dann der Fall ist, wenn die Sequenz vermehrt geladene oder stark polare funktionelle Gruppen enthält oder wenn die Sekundärstruktur des Proteins Schleifen aufweist, welche bevorzugt aus dem Molekül herausragen. Diese Bedingung ist meist dann erfüllt, wenn beispielsweise Asparagin (Asn), Asparaginsäure (Asp), Prolin (Pro), Glutamin (Gln), Glutaminsäure (Glu) und/oder Glycin (Gly) vermehrt in der betrachteten Sequenz vorkommen.
b) Die Zahl der potentiellen Epitope auf dem ausgewählten Peptidbruchstück soll einerseits möglichst klein sein, andererseits soll das Peptidbruchstück aber groß genug für eine Immunantwort sein. Die ausgewählte Sequenz sollte 20, bevorzugt 12, besonders bevorzugt 10, ganz besonders bevorzugt 8 Aminosäuren nicht überschreiten und nicht kürzer sein als 4, bevorzugt 5, besonders bevorzugt 6 Aminosäuren. Bewährt haben sich Peptidbruchstücke mit 6-13, bevorzugt 7-11, insbesondere 8-10 Aminosäuren.
c) Bevorzugt sollte die ausgewählte Sequenz nicht am N- oder C-Terminus des betreffenden nativen Proteins liegen, wobei unter N- und C-Terminus hier die entsprechenden Termini des gesamten nativen Proteins verstanden werden. Ist dieses gesamte Protein aus mehreren miteinander verknüpften Proteinen aufgebaut, so kann die Sequenz selbstverständlich an einem innenliegenden N- oder C- Terminus dieses integrierten Proteins liegen.

### 2. Herstellung der betreffenden Aminosäuresequenz

Zur Herstellung des ausgewählten Proteinbruchstückes bietet sich beispielsweise die literaturbekannte Peptidsynthese nach Merrifield an. Es ist aber auch durchaus möglich, geeignete Fragmente aus einer enzymatischen oder chemischen Spaltung des nativen Proteins zu erhalten. Kurze Sequenzen können auch rein chemisch synthetisiert werden.

### 3. Sofern erforderlich Ankopplung eines Carriers

Insbesondere bei kurzen Proteinfragmenten, die selbst gar keine oder nur eine unzureichende Immunantwort provozieren, aber auch bei immunogenen Fragmenten empfiehlt es sich, einen Carrier an das ausgewählte Proteinbruchstück zu koppeln. Diese Ankopplung geschieht nach Verfahren, die dem Fachmann bekannt sind, beispielsweise über Kopplungsreagenzien wie Glutaraldehyd oder 1-Hydroxy-2-nitrobenzol-4-sulfonsäure-N-maleinimido-6-caproyl-ester-natriumsalz (mal-sac-HNSA). Als Carrier können beispielsweise eingesetzt werden: Polymere wie Polyethylenglykol, Polyacrylamid oder Poly-d-Glutamin-d-Lysin oder Fettsäurederivate wie PAM-3-Cys (PAM=Palmitoyl) oder Proteine wie Rinderserumalbumin (BSA) oder Keyhole Limpet Hemocyanin (KLH).

Bevorzugt werden mehrere Moleküle des Proteinfragments an den Carrier gekoppelt.

### 4. Immunisierung einer Spezies mit dem Proteinfragment bzw. mit dem carrier-gebundenen Proteinfragment

Die Immunisierung einer Spezies mit dem Proteinbruchstück bzw. mit dem carrier-gebundenen Proteinbruchstück geschieht nach literaturbekannten Verfahren, beispielsweise durch intramuskuläre Injektion des Immunogens, gegebenenfalls zusammen mit einem Adjuvans wie KFA (komplettes Freundsches Adjuvans) oder IFA (inkomplettes Freundsches Adjuvans). Sofern erforderlich, kann nach Erhalt der Immunantwort ein- oder mehrmals "geboostert" werden. Die Auswahl der Spezies ist nicht kritisch, beispielsweise eignen sich Mäuse, Ratten, Kaninchen, Schafe oder Ziegen. Zwecks Herstellung größerer Mengen an antikörper-enthaltenden Seren ist es jedoch vorteilhaft, größere Tiere, wie Schafe oder Ziegen, einzusetzen.

### 5. Gewinnung der Antikörper aus den Seren

Prinzipiell nach Erhalt der ersten Immunantwort kann das Antiserum abgenommen werden. Je nach verwendeter Tierspezies erhält man höhere Titer jedoch erst nach ein- oder mehrmaligem Boostern mit dem entsprechenden Immunogen. Je nach Verwendungszweck wird das Serum gereinigt und angereichert oder aber direkt ohne weitere Reinigung im Assay-Medium verdünnt und eingesetzt. Insbesondere für die Herstellung von Sandwichassays empfiehlt sich die Reinigung und Anreicherung des Serums. Dies kann beispielsweise durch Ammoniumsulfatfällung und anschließende Auftrennung auf einer Affinitätssäule erfolgen, auf der ein entsprechendes Antigen immobilisiert ist. Hierbei werden alle Proteine abgetrennt, welche mit dem immobilisierten Protein keine Wechselwirkung zeigen. Die Antikörper, die das betreffende Protein erkennen - und somit an dem immobilisierten Protein gebunden sind - können dann von der Säule eluiert werden.

Alternativ zu der oben unter 5. beschriebenen Methode der Gewinnung polyklonaler Antikörper können natürlich auch monoklonale Antikörper hergestellt werden. Dies geschieht beispielsweise durch Immunisierung von Mäusen, wie oben unter 4. beschrieben und anschließender Fusion der Mäusemilzzellen beispielsweise mit NS 1 Myelomzellen und Klonierung von geeigneten Zellen. Gegebenenfalls können die so gewonnenen monoklonalen Antikörper beispielsweise durch Injektion in Nacktmäuse vermehrt werden. Prinzipiell ist die Herstellung solcher monoklonalen Antikörper dem Fachmann bekannt und in der Literatur beschrieben. Die Aufarbeitung und Reinigung kann dann wie oben unter 5. beschrieben, erfolgen.

Die erfindungsgemäßen Antikörper können zur Herstellung von Immunoassays verwendet werden. Bei solchen Immunoassays können beispielsweise die erfindungsgemäßen Antikörper oder ein Antigen auf einer Festphase immobilisiert sein. Verfahren zur Immobilisierung von Antigenen und Antikörpern auf Festphasen wie synthetischen oder natürlichen Polymeren wie Polystyrol, Polypropylen, PVC oder Latex in verschiedenen geometrischen Ausführungsformen, wie Röhrchen, Kugeln oder Mikrotitrationsplatten sind dem Fachmann bekannt Der Immunoassay kann beispielsweise ein kompetitiver Assay oder ein Sandwichassay sein. In beiden Fällen ist ein Bestandteil - entweder das Antigen oder der Antikörper - zum Zwecke der Detektion markiert. Die Markierung erfolgt meist über ein radioaktives, chemilumineszentes oder enzymatisches Label. Auch solche Verfahren zur Markierung von Antigenen und Antikörpern sind dem Fachmann bekannt. Da die erfindungsgemäßen Antikörper in der Lage sind, sowohl die nativen Proteine einer Spezies als auch die korrespondierenden nativen Proteine anderer Spezies und sogar Derivate, Fragmente, synthetische und natürliche Vorstufen oder denaturierte Produkte dieser Proteine - sofern sie das zur Immunisierung verwendete Peptidbruchstück oder zumindest Unterbruchstücke davon, die mindestens 60-80 % des zur Immunisierung verwendeten Peptidbruchstücks entsprechen, aufweisen - zu erkennen, ist es vorteilhaft, zur Herstellung eines Multi-Spezies-lmmunoassays die erfindungsgemäßen Antikörper zu markieren und damit nach literaturbekannten Verfahren einen RIA(Radioimmunoassay), CIA/LIA ((Chemi)-Lumineszenzimmunoassay) oder EIA (Enzymimmunoassay) aufzubauen.

Als besonders vorteilhaft für die Bestimmung von gentechnologisch hergestellten Produkten, welche in Mikroorganismen als schwerlösliche "Inclusion bodies" anfallen, in einem RIA, hat sich ein Puffersystem erwiesen, welches neben üblichen Puffersystemen wie Phosphatpuffer (Na₂HPO₄, NaH₂PO₄), Tris-Puffer (Tris(hydroxymethyl)aminomethan) oder Barbiturat-Puffer (beispielsweise Natriumdiethylbarbiturat), mindestens ein Protein wie Rinderserumalbumin (BSA), Milcheiweiß, Ovalbumin, Eiereiweiß, Trockenmilchpulver oder Gelatine und mindestens ein ionisches Detergens wie Natriumdodecylsulfat (SDS), Hexadecyltrimethylammoniumbromid oder ein Gallensalz und/oder mindestens ein nicht-ionisches Detergens wie ®Nonidet P40, ®Triton X100 oder ®Tween 20 enthält.

In einer besonderen Ausgestaltung betrifft die Erfindung Antikörper, welche sowohl mit Insulinen verschiedener Spezies als auch mit Insulinderivaten, Fragmenten, synthetischen und natürlichen denaturierten InsulinVorstufen und Derivaten dieser denaturierten Insulin-Vorstufen Immunkomplexe bilden. Zur Herstellung dieser "Multi-Spezies-Insulin-Antikörper" wird als Immunogen ein Insulinfragmentgemäß obengenannter Kriterien 1a-b ausgewählt. Geeignet sind beispielsweise die Sequenzen A₁ - A₇ oder A₁₁ - A₁₇ oder A₁₁ - A₂₁ der Insulin-A-Kette sowie die Regionen um die Cysteine der B-Kette. Als besonders geeignet hat sich das Insulin-A-Kette-(14-21)-Octapeptid Tyr - Glu - Leu - Glu - Asn - Tyr - Cys - Asn erwiesen. Dieses Octapeptid kann nach literaturbekannten Verfahren (W. König, K. Kernebeck, Liebigs Ann. Chem., 1979, 227-247) hergestellt werden. Die Carrierankopplung, Immunisierung und Antikörpergewinnung erfolgt nach den oben angegebenen Verfahrensschritten 3-5. Auch ohne zusätzliche Aufarbeitung und Reinigung können die erhaltenen Insulin-Antikörper zur Herstellung eines Multi-Spezies-lnsulin-Assays verwendet werden.

Ein solcher Multi-Spezies-Insulin-Assay kann beispielsweise als RIA, CIA/LIA oder EIA nach literaturbekannten Verfahren aufgebaut werden. Dabei können die erfindungsgemäßen Insulin-Antikörper sowohl in freier Lösung (beispielsweise in einem Fällungs-RIA) oder an einer Festphase gebunden (immobilisiert) vorliegen. Für den Fällungs-RIA eignen sich dann beispielsweise radioaktiv, bevorzugt Radiojod markierte Insuline, Insulinfragmente, Insulinderivate, natürliche oder synthetische Insulinvorstufen oder beispielsweise das radioaktiv markierte Peptidbruchstück, welches zur Generierung der erfindungsgemäßen Insulin-Antikörper verwendet wurde, insbesondere das Radiojod markierte Insulin-A-Kette (14-21)-Octapeptid. Für die Herstellung eines Sandwich-lmmunoassays werden zwei Antikörper verwendet, von denen einer - meist der nicht-Festphasen gebundene - markiert ist. Die beiden Antikörper können gegen das gleiche Epitop des Insulins gerichtet sein, bevorzugt sind sie jedoch gegen unterschiedliche Epitope des Insulins gerichtet. Für solch einen Sandwich Immunoassay, bei dem die beiden verwendeten Antikörper gegen verschiedene Epitope gerichtet sind, verwendet man bevorzugt polyklonale oder monoklonale, affinitätsgereinigte, Radiojod markierte Antikörper. Die Markierung der Antikörper bzw. Antigene (Insulin, Insulinfragmente, Insulinderivate, natürliche oder synthetische Vorstufen) erfolgt nach literaturbekannten Verfahren, beispielsweise kann für die Radiojodmarkierung die Iodo-Gen-Methode angewandt werden.

Der erfindungsgemäße Multi-Spezies-Insulin-Assay weist gegenüber Insulinassays gemäß Stand der Technik den Vorteil auf, daß mit ihm sowohl Insuline verschiedener Spezies, als auch Insulinderivate, Insulinfragmente, synthetische und natürliche denaturierte Insulinvorstufen und Derivate dieser denaturierten Insulinvorstufen gemessen und bestimmt werden können.

Es hat sich gezeigt, daß sogar solche Proteine nachgewiesen und bestimmt werden können, welche Aminosäuresequenzen enthalten, die nur 60-80 % des Peptidbruchstücks darstellen, welches zur Immunisierung (und damit zur Antikörpergenerierung) verwendet wurde. So lassen sich beispielsweise mit dem Multi Spezies-lnsulin-Assay, welcher Antikörper enthält, die durch Immunisierung mit dem Insulin-A-Kette (14-21)-Octapeptid erhalten wurden, auch solche Proteine bestimmen, welche nur das Hexapeptid (16-21) enthalten. Folgende Insuline, Insulinderivate oder vom Insulin abgeleitete Proteine können beispielsweise mit dem erfindungsgemäßen Multi-Spezies Insulinassay bestimmt werden:
1. β-Galaktosidase-Insulin-Fusionen expremiert in E.coli P1, P6, P1-Trimer-Des-Met-Des-Cys, P1-Trimer-Des-Met, P1-Poly-Gly-Des-Met, P1-Poly-Gly-Des-Met-Des-Cys, P-Lz-gamma;
2. Interleukin-2 Insulin-Fusionen expremiert in E.coli pB40, pK52, pGF12, pIK10, pSW3, pSW2, pSW3*M;
3. trp-Insulin-Fusionen expremiert in E.coli pB70, plNT 14, plNT 30, plNT 41, pSL 27, plNT 91;
4. Insuline verschiedener Spezies Human-Insulin, Schweine-Insulin, Schaf-Insulin, Pferd-Insulin, Rind- Insulin, Huhn-lnsulin, Ente-Insulin, Truthahn-lnsulin, Gans- Insulin, Alligator-Insulin, Klapperschlange-Insulin, Colubrid-Snake-lnsulin, Sei Whale-lnsulin, Elefant-Insulin, Ziege-Insulin, Hund-Insulin, Affe-Insulin, Sperm Whale-Insulin, Fin Whale-lnsulin, Ratte- Insulin, Hamster-Insulin, Kaninchen-Insulin;
5. Insulinderivate
   B31-Mono-Arg-Human-lnsulin, B31,B32-Di-Arg-Human-lnsulin, B1-Des-Phe-Schweine-Insulin, A14-Monojodo-Human-Insulin;
6. Insulin-Fragmente
   Insulin-A-Kette-Tetrasulfonat (Rind), Insulin-A-Kette-Tetrasulfonat (Mensch), Insulin-A-Kette-(14-21)Octapeptid, Insulin-A-Kette-(16-21)-Hexapeptd;
7. Insulin-Vorstufen
   Prä-Pro-Insulin-S-Sulfonat (P1), Prä-Pro-Insulin (P1), Prä-Pro-Insulin (pSW 3), Pro-Insulin (Schwein);

Weiterhin weist der erfindungsgemäße Multi-Spezies-Insulin-Aassay den Vorteil auf, daß auch in Gegenwart von ionischen und nicht-ionischen Detergenzien, Hilfsproteinen, Detergenzmischungen sowie Detergenz-Hilfsprotein Mischungen gemessen werden kann. Als Detergenzien können beispielsweise Natriumdodecylsulfat (SDS), ®Triton X 100 oder ®Nonidet P 40 und als Hilfsproteine Rinderserumalbumin (BSA), Hühnereiweiß, Ovalbumin oder E.coli-Proteine eingesetzt werden. Ionische Detergenzien können bevorzugt im Bereich von 0-0,3%, nichtionische Detergenzien bevorzugt im Bereich von 0-2 % und Hilfsproteine bevorzugt im Bereich von 0-3 % eingesetzt werden (Prozentangaben in w/v = weight/volume). Die Messung in Gegenwart dieser Substanzen hat den Vorteil, daß auch beispielsweise schwerlösliche Produkte aus gentechnologischer Herstellung der Messung zugänglich sind, ohne den Assay wesentlich zu stören, was mit immunometrischen Verfahren gemäß Stand der Technik bisher nicht möglich war. Weder Hilfsproteine oder Fremdproteine wie Calcitonin oder das Nonapeptid Buserelin, noch übliche Puffersysteme stören den Multi-Spezies Assay wesentlich. So hat sich beispielsweise für die radioimmunologische Bestimmung (RIA-Bestimmung) von gentechnologisch hergestellten Produkten, welche in Mikroorganismen als schwerlösliche "Inclusion bodies" anfallen, ein Puffersystem als sehr geeignet erwiesen, welches neben den üblichen Puffersubstanzen wie Phosphatpuffer (Na₂HPO₄, NaH₂PO₄), Tris-Puffer (Tris(hydroxymethyl)aminomethan) oder Barbiturat-Puffer (beispielsweise Natriumdiethylbarbiturat), mindestens ein Protein wie Rinderserumalbumin (BSA), Milcheiweiß, oder Ovalbumin und mindestens ein ionisches Detergens wie Natriumdodecylsulfat (SDS), Hexadecyltrimethylammoniumbromid oder ein Gallensalz und/oder mindestens ein nicht-ionisches Detergens wie ®Nonidet P40, ®Triton X100 oder ®Tween 20 enthält.

Da die erfindungsgemäßen Insulin-Antikörper zur gleichwertigen Erfassung von erheblich unterschiedlichen antigenen Insulinen herangezogen werden können, eignen sie sich auch in Kombination mit bisher bekannten hochspezifischen Insulin Antikörpern zur Untersuchung der Tertiärstruktur und der Lokalisierung von essentiellen und weniger essentiellen Strukturmerkmalen im Insulinmolekül.

### Beispiele

### Beispiel 1

### Kopplung von Insulin-A-Kette (14-21)-Octapeptid an BSA

Das geschützte Insulin-A-Kette (14-21)-Octapeptid wird nach W. König, K. Kernebeck, Liebigs Ann. Chem. 1979, 227-247 synthetisiert. Zur Konjugation an BSA als Carrier-Molekül wird das geschützte Insulin-A-Kette (14-21)-Octapeptid Ddz-Tyr(tBu)-Gln-Leu-Glu(OtBu)-Asn-Tyr(tBu)-Cys(Trt)-AsnOtBu durch Behandlung mit einer Mischung aus Trifluoressigsäure und Ethanthiol aller Schutzgruppen entledigt (nach W. König, K. Kernebeck, Liebigs Ann. Chem., 1979, 227-247). Das resultierende Produkt wird dann mit Hilfe des bifunktionellen Kupplungsreagenzes 1-Hydroxy-2-nitrobenzol-4-sulfonsäure-N-maleinimido-6-caproyl-ester Natriumsalz (mal-sac-HNSA) an BSA kovalent gebunden. Dafür werden 55 mg mal-sac HNSA zu einer Lösung von 111 mg BSA (entspricht 95 Äquivalenten Lysin) in 10 ml 0,1 molarem Phosphatpuffer bei pH 7,4 gegeben. Nach 60 Minuten Rühren bei Raumtemperatur wird die Reaktionsmischung über Sephadex G 25 in 0,1 molarem Phosphatpuffer bei pH 6,2 chromatographiert und der zuerst eluierende Peak gesammelt. Zu dieser Lösung werden 67 mg (65 µmol) Insulin-A-Kette (14-21)-Octapeptid gegeben. Danach läßt man über Nacht bei Raumtemperatur stehen. Die Reaktionsmischung wird nun gegen Wasser dialysiert und die resultierende Lösung lyophylisiert.

| | |
|---|---|
| Ausbeute | 122 mg |
| Proteingehalt | 83 % |

15 Moleküle Octapeptid pro Molekül BSA (bestimmt durch Aminosäureanalyse)

### Beispiel 2

### Immunisierung

Zur Immunisierung wurden drei Tierarten verwendet, und zwar mischrassige Hauskaninchen (Zahl der Individuen = 3) und je ein Schaf und eine Ziege. Die Immunisierung wurde bei allen Tieren gleichzeitig begonnen, wobei den Kaninchen je 0,1 mg des Octapeptid/BSA-Konjugats aus Beispiel 1 in KFA(komplettes Freundsches Adjuvants, Difco) und Schaf und Ziege je 2,5 mg des Octapeptid/BSA-Konjugats in KFA als Initialdosis intramuskulär appliziert wurde. In der dritten Woche nach Erstapplikation wurde mit der gleichen Menge Octapeptid/BSA-Konjugat in IFA (Inkomplettes Freundsches Adjuvans, Behring) geboostert und dieser Vorgang in der 4., 8., 13., 18. und 25. Woche wiederholt. In der 27., 32. und 37. Woche wurde mit der gleichen Menge an reinem nicht-BSA-konjugiertem Octapeptid in IFA geboostert. Die Abnahme der Antiseren erfolgte jeweils zum ersten Mal in der 10. Woche und dann jeweils zehn Tage nach dem Boostern. Die Titerbestimmung erfolgte wie in der Literatur beschrieben (T. Chard, "An introduction to Radioimmunoassay and Related Techniques", Elsevier Science Publishers, Amsterdam (1987), S. 101-102). Dabei wurde eine Verdünnungsreihe (1:10 - 1:10⁶) des abgenommenen Serums in MSTB-Puffer (Zusammensetzung des Puffers s. Beispiel 3) angesetzt und die Menge an jeweils gebundenem Tracer unterAssaybedingungen (s. Beispiel 3) bestimmt. Der Titer ist dann derjenige Wert, bei der die Antikörper des jeweiligen Serums bei einer gegebenen Verdünnung 50 % des eingesetzten Tracers binden. Der Titer wird als reziproker Wert angegeben. Die Seren der oben beschriebenen immunisierten Tiere hatten Titer von 1:500 - 1:10000.

### Beispiel 3

### Herstellung und Durchführung eines Radioimmunoassays Verwendete Materialien

### Antiserum

Zur Herstellung eines Radioimmunoassays wurde ein Schafantiserum (S 239) mit einem Titer von 1:500 verwendet. Das eingesetzte Antiserum wurde ohne weitere Reinigung direkt eingesetzt. Die Verdünnung des Serums betrug 1:20 (in MSTB-Puffer); es wurde bei -20°C gelagert. Die Gebrauchsverdünnung betrug 1:500 (in MSTB-Puffer).

### MSTB-Puffer

Der verwendete MSTB-Puffer bestand aus:

| | |
|---|---|
| 0.1 M | Morpholinopropansulfonsäure (MOPS) eingestellt auf pH 7.5 mit 1M NaOH |
| 2,5 % | (w/v) Rinderserumalbumin |
| 0,2 % | (w/v) Natriumdodecylsulfat |
| 0,2 % | (w/v) ®Triton X-100 |
| 0,04% | (w/v) Natriumazid |

### Immunglobulinlösung

Zur Assaydurchführung wurde eine Immunglobulinlösung in einer Konzentration von 10 mglml bidest. Wasser verwendet.

### Tracer

Als Tracer wurde ¹²⁵J-markiertes Schweineinsulin (Behringwerke AG, Marburg, Prod.-Nr. OCSM) verwendet (10 ng < 74 KBq Lyophilisat).

Pro Teströhrchen wurde eine Totalaktivität von 20.000-30.000 counts eingesetzt

### Standards

Die Standards wurden zunächst auf ihren Proteingehalt untersucht Anschließend wurde der Gehalt an später im RIA zu bestimmender Substanz (Insuline verschiedener Spezies, 16 Insulinderivate, Insulinvorstufen etc.) festgestellt. Dann wurden die Standards auf eine Konzentration von 2000 ng/ml in MSTB-Puffer eingestellt. Für die Aufnahme der Standardkurven wurde jeweils eine geometrische Verdünnungsreihe in folgenden Konzentrationen (Angaben in ng/ml MSTB-Puffer) hergestellt: 3.71; 7.5; 15; 30; 60; 120; 240; 480; 960; 1920.

### Aufnahme der Standardkurven (Assaybedingungen)

Zur Ermittlung der Standardkurven wurden jeweils 100 µl Standard, 100 µl Tracer und 100 µl Antiserum in ein Röhrchen (Fa. Sarstedt, Best-Nr. 55-535) pipettiert. Die Probe wurde gut durchmischt und über Nacht (18 Stunden) bei Raumtemperatur (18-25°C) stehengelassen. Vor dem Ausfällen mit 1000 µl Polyethylenglykol (Molekulargewicht ca. 4000) wurde mit 50 µl Immunglobulinlösung versetzt und gut durchmischt. Nach 20 Minuten wurde mit 1500 x g abzentrifugiert und der Überstand abdekantiert. Das Präzipitat wurde dann im Gamma-Counter (Gamma-Counter 1277, Pharmacia LKB) 1 Minute gemessen. Es kam jeweils eine Doppelbestimmung zur Auswertung.

### Ermittlung des Leerwerts

Zur Ermittlung des Leerwerts wurde wie oben unter "Aufnahme der Standardkurven" beschrieben, verfahren. Anstelle des Standards wurde hier jedoch 100 µl MSTB-Puffer eingesetzt.

### Beispiel 4

### Messung verschiedener Insuline und davon abgeleiteter Proteine im RIA

Anhand der Standards mit zuvor bestimmtem Gehalt an Insulin bzw. vom Insulin abgeleitetem Protein (s. Beispiel 3: Standards), wurden für folgende Insuline Standardkurven aufgenommen:
Humaninsulin (Fig. 1). Schweineinsulin (Fig. 2), Rinderinsulin (Fig. 3), Schafinsulin (Fig. 4), Hühnerinsulin (Fig. 5) und Pferdeinsulin (Fig. 6), die Derivate Des-Phe-B1-Schweineinsulin (Fig. 7), Di-Arg-B31-B32-Humaninsulin (Fig. 8), Mono-Arg-B31-Humaninsulin (Fig. 9), Pro-Schweineinsulin (Fig. 10) sowie Insulin-A-Kette-TetraSulfonat (Fig. 11) und (14-21)-Octapeptid (Fig. 12) und (16-21)-Hexapeptid (Fig. 13).

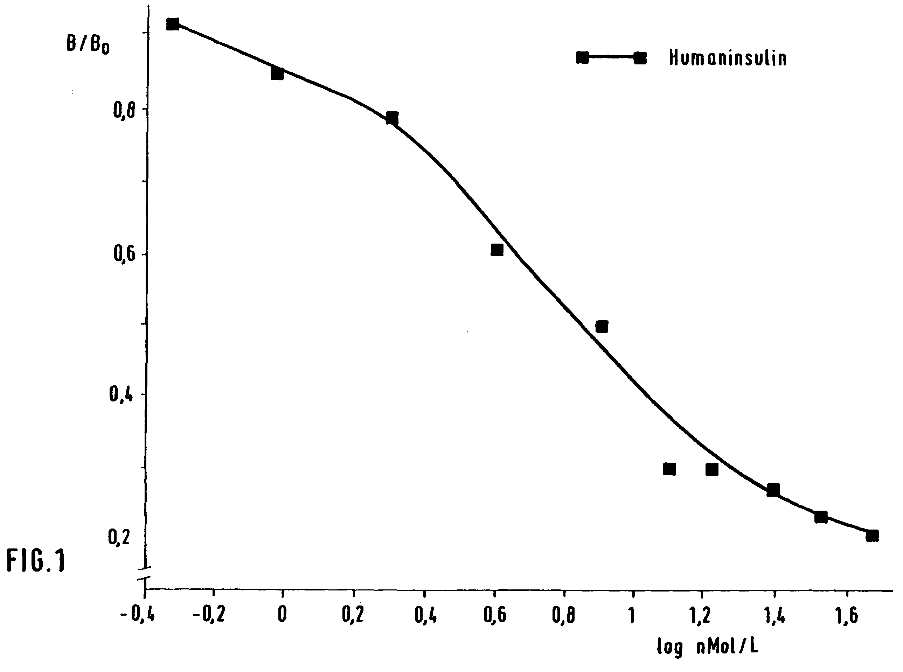

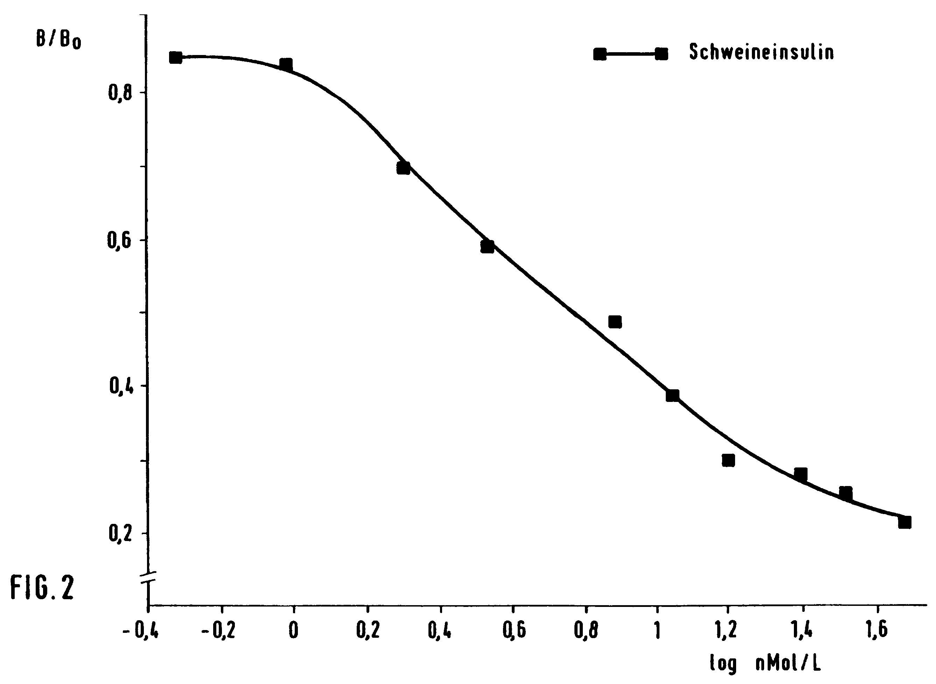

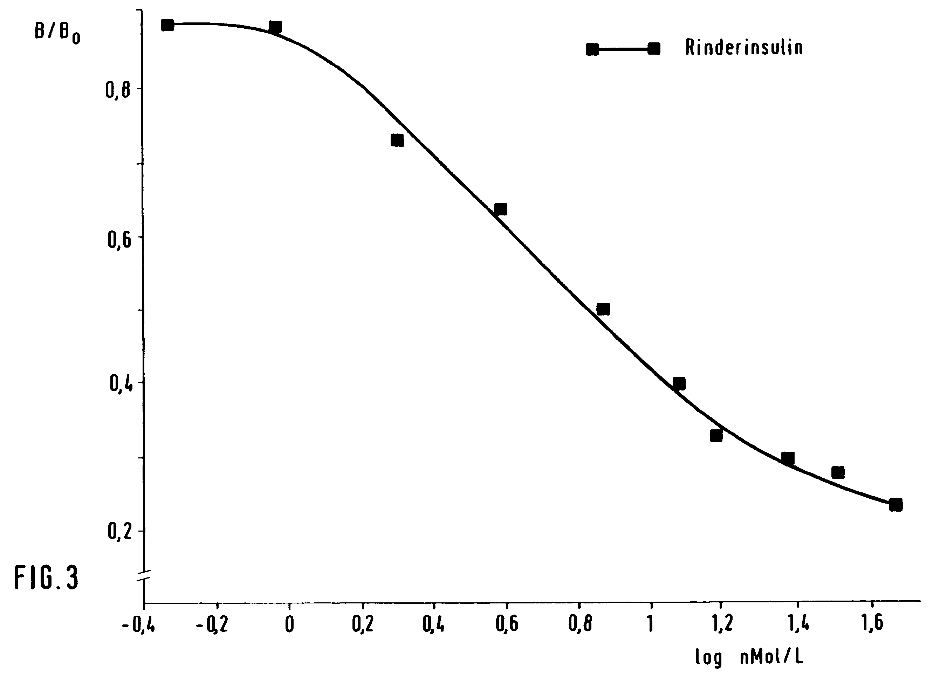

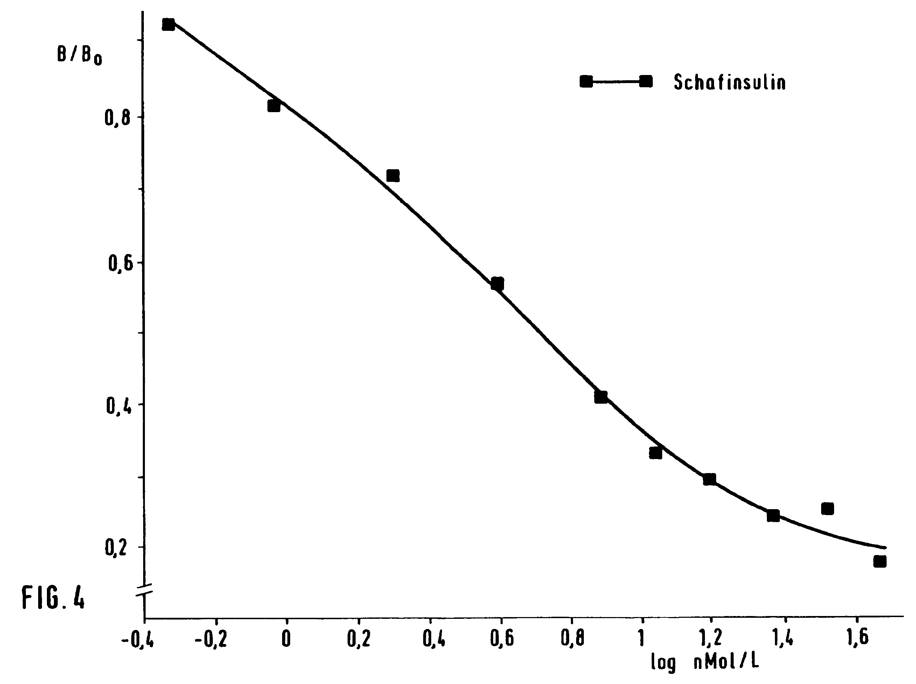

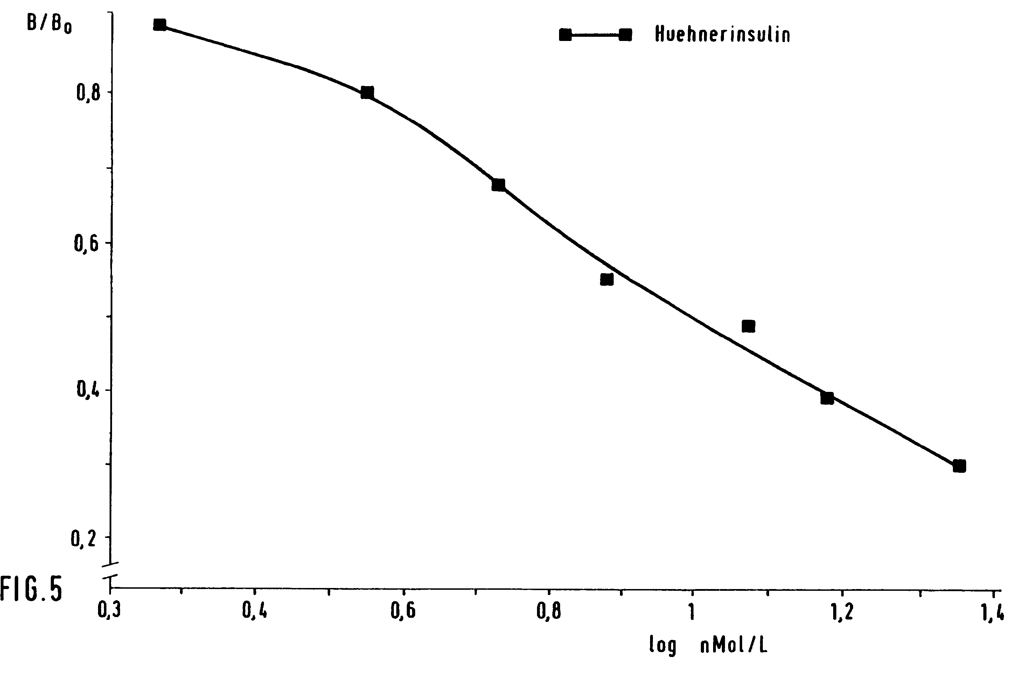

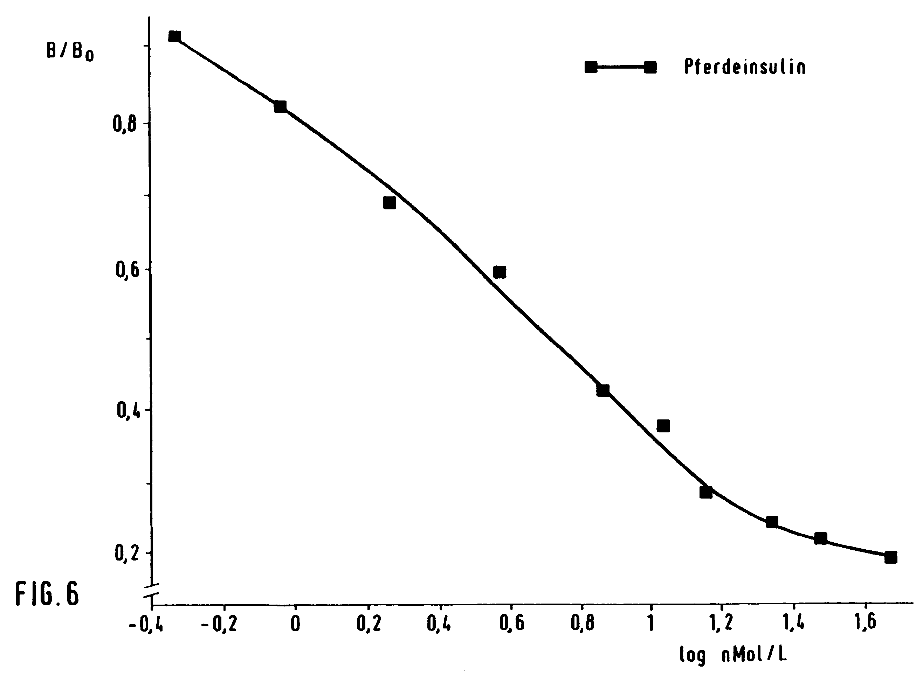

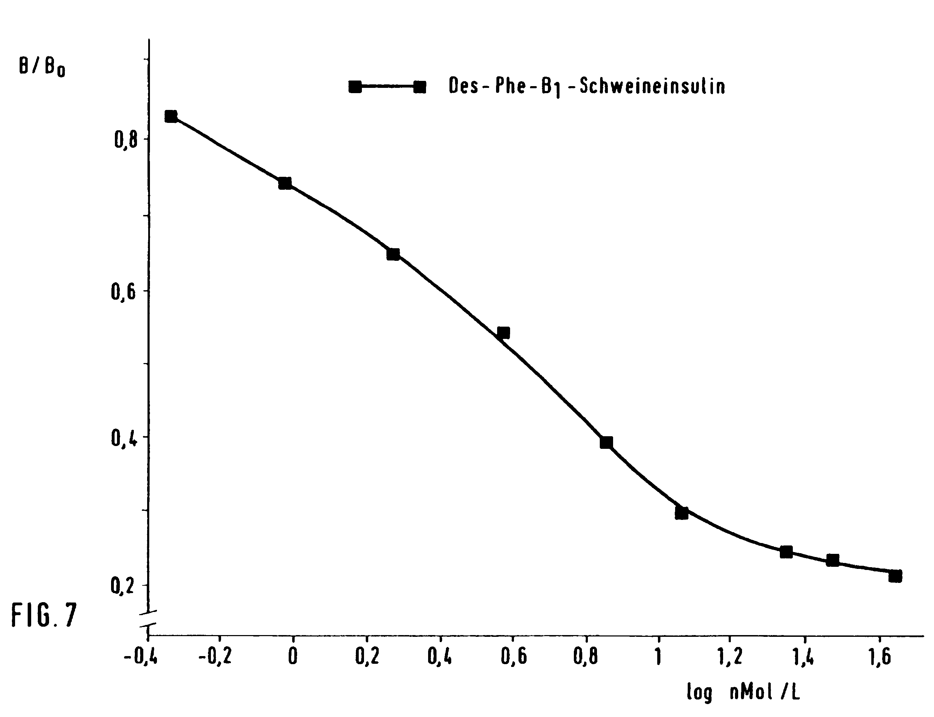

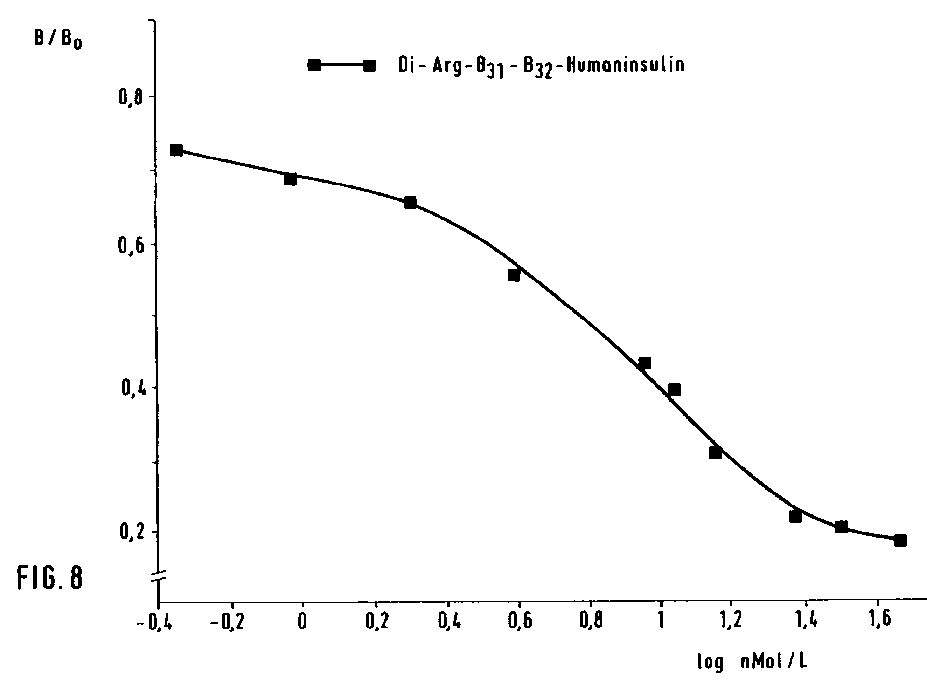

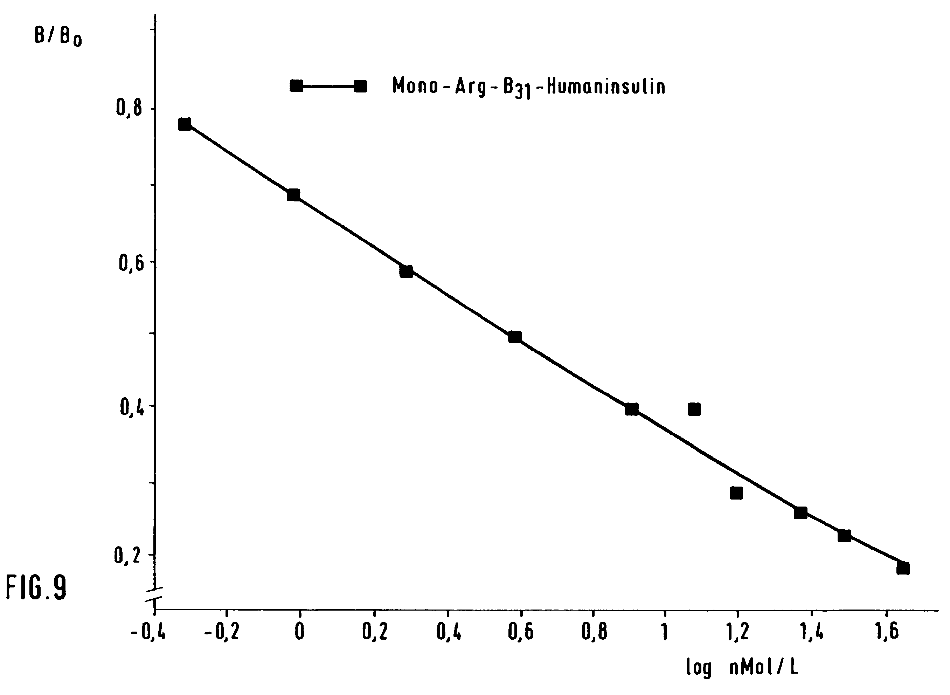

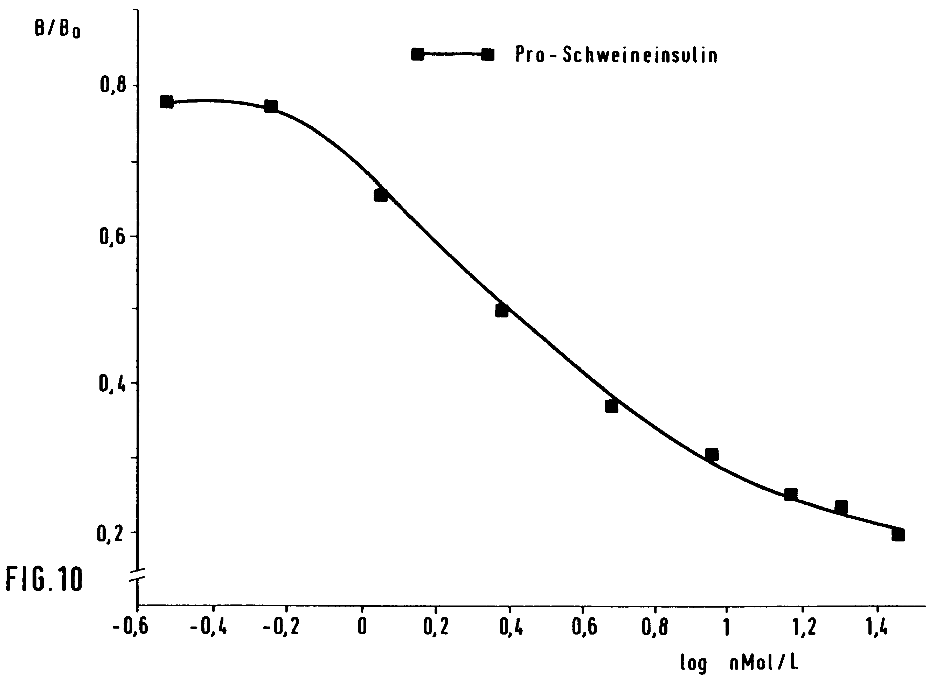

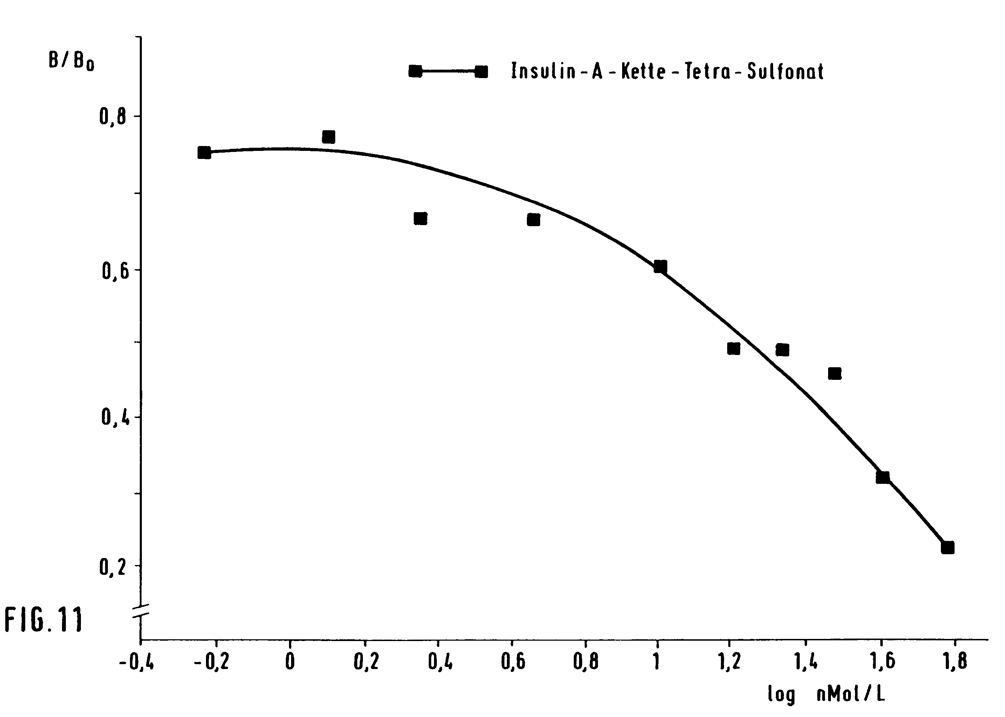

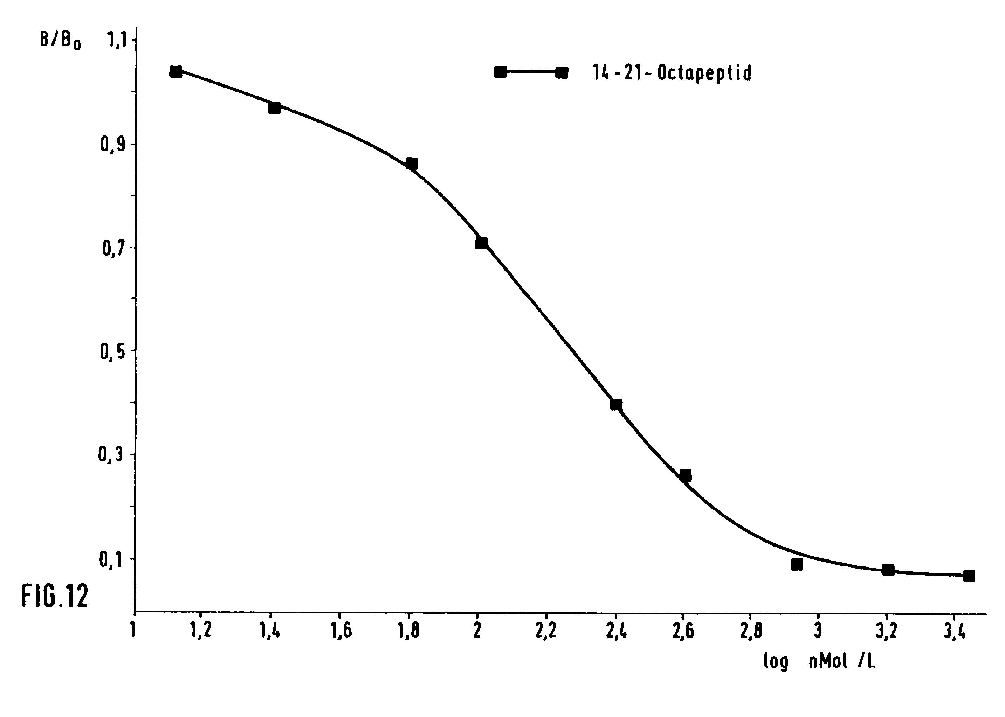

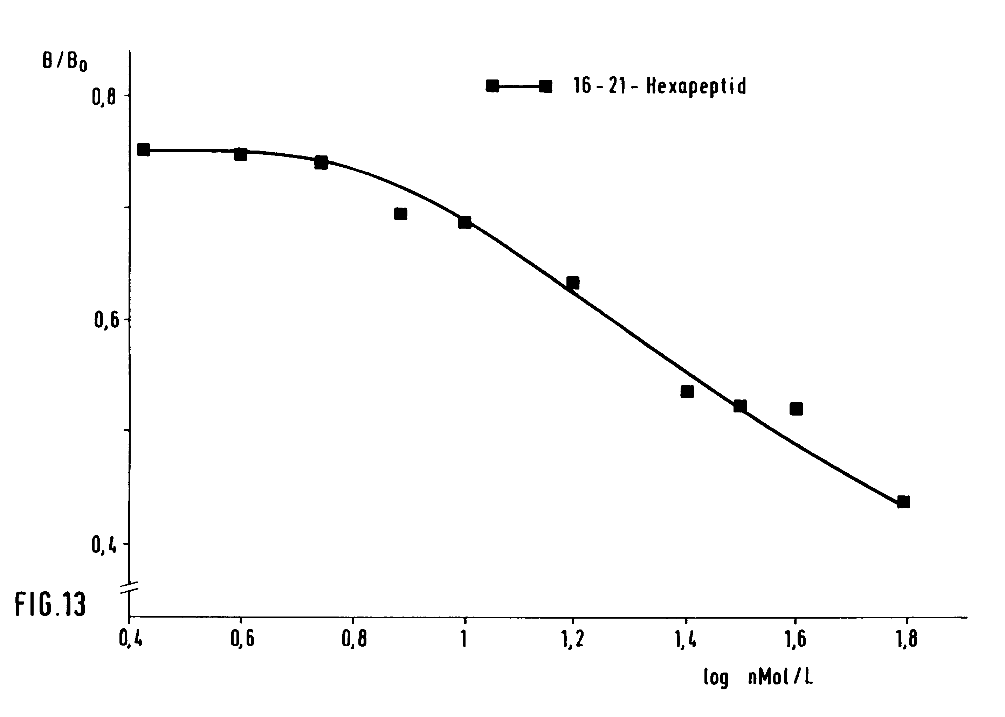

Die in den Figuren angegebenen Werte B/B₀ geben den Quotienten aus gemessener Aktivität B und maximaler Aktivität B₀ (komplette Sättigung des Antikörpers mit Tracer) an. Die in den Figuren 1 - 13 dargestellten Standardkurven belegen deutlich, daß mit dem erfindungsgemäßen RIA unter verwendung der erfindungsgemäßen Antikörper eine empfindliche Nachweismethode für eine Vielzahl von Insulinen zur Verfügung gestellt wurde.

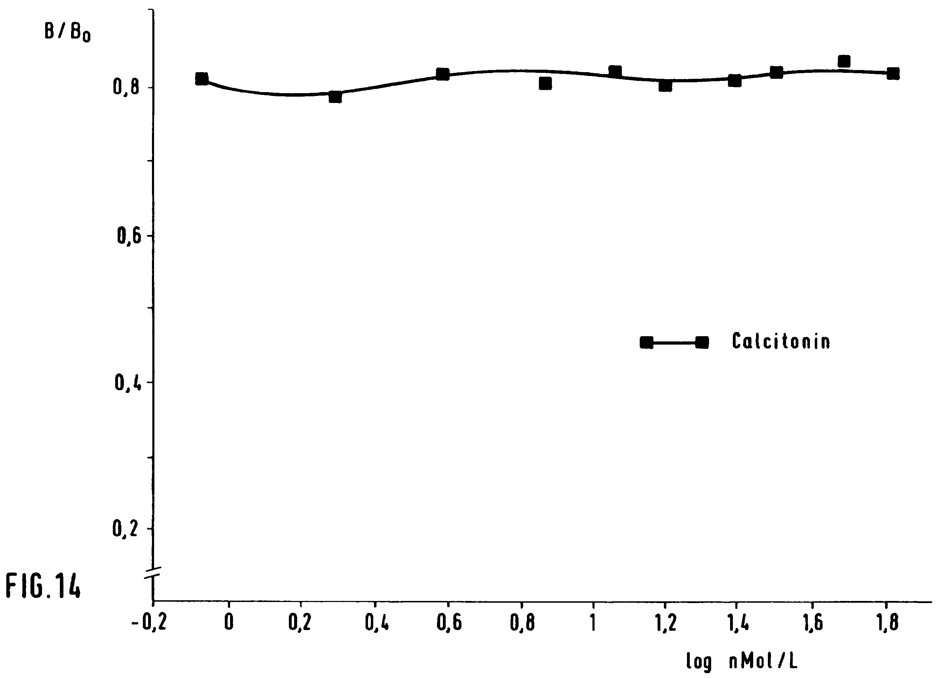

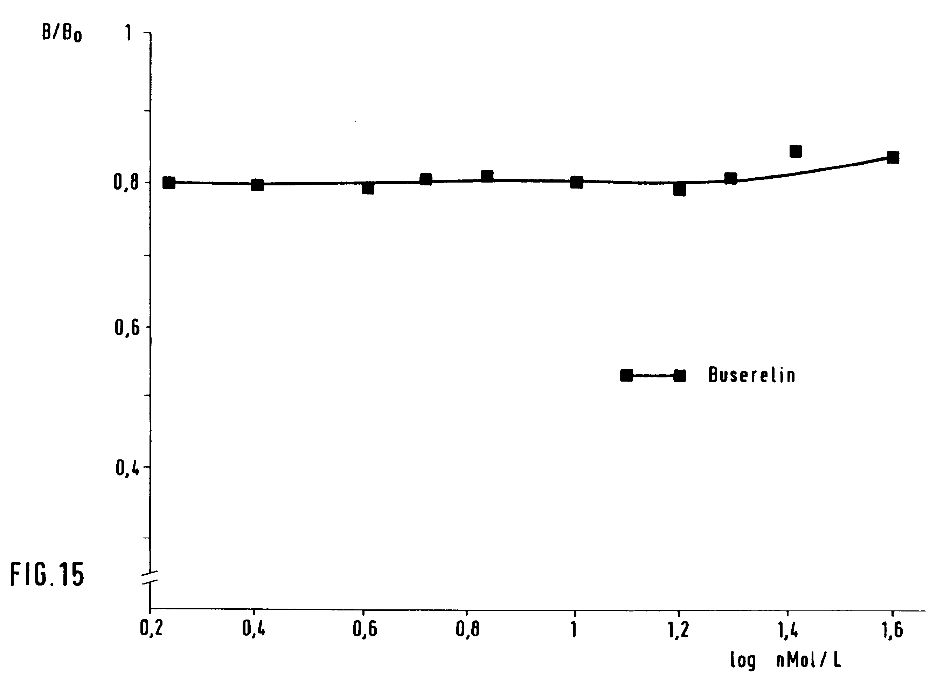

Der Einfluß von Fremdproteinen und des Puffersystems auf die Messungen wurde untersucht und es wurde festgestellt, daß weder hohe Konzentrationen an BSA noch an E.coli-Proteinen den Assay stören. Als negativ Kontrolle um den Nachweis einer fehlenden Kreuzreaktivität mit kleinen Peptid Strukturen zu erbringen, wurden Calcitonin und Buserelin in gleichen Konzentrationen wie das Octapeptid dem Testansatz zugesetzt. Es zeigte sich keine Kreuzreaktivität (s. Fig. 14 und Fig. 15).

## Patentansprüche (Patentansprüche für folgende(n) Vertragsstaat(en): AT, BE, CH, DE, DK, FR, GB, IT, LI, LU, NL, SE)

1. Antikörper, dadurch gekennzeichnet, daß sie durch Immunisierung mit einem Peptidbruchstück, welches eine hochkonservierte Aminosäuresequenz des nativen Proteins Insulin darstellt, erhalten werden.

2. Antikörper nach Anspruch 1, dadurch gekennzeichnet, daß die Aminosäuresequenz sich an der Oberfläche des nativen Proteins befindet.

3. Antikörper nach Anspruch 1 oder 2, dadurch gekennzeichnet, daß die Aminosäuresequenz geladene und/oder stark polare funktionelle Gruppen enthält.

4. Antikörper nach einem oder mehreren der Ansprüche 1 bis 3, dadurch gekennzeichnet, daß die Aminosäuresequenz mindestens eine Aminosäure enthält, ausgewählt aus: Asn, Asp, Pro, Gly, Gln, Glu.

5. Antikörper nach einem oder mehreren der Ansprüche 1 bis 4, dadurch gekennzeichnet, daß die Aminosäuresequenz 6 - 13 Aminosäuren lang ist.

6. Antikörper nach Anspruch 5, dadurch gekennzeichnet, daß die Aminosäuresequenz 8 Aminosäuren nicht überschreitet und nicht kürzer als 6 Aminosäuren ist.

7. Antikörper nach einem oder mehreren der Ansprüche 1 bis 6, dadurch gekennzeichnet, daß die Aminosäuresequenz in der Insulin-A- oder -B-Kette liegt.

8. Antikörper nach einem oder mehreren der Ansprüche 1 bis 7, dadurch gekennzeichnet, daß das Peptidbruchstück das Insulin-A-Kette(14-21)-Octapeptid ist.

9. Antikörper nach einem oder mehreren der Ansprüche 1 bis 8, dadurch gekennzeichnet, daß sie kreuzreaktiv sind mit Proteinen, welche Aminosäuresequenzen enthalten, die mindestens zu 60 - 80 % mit der Aminosäuresequenz des zur Immunisierung eingesetzten Peptidbruchstücks übereinstimmen.

10. Antikörper nach einem oder mehreren der Ansprüche 6 bis 9, dadurch gekennzeichnet, daß sie kreuzreaktiv sind mit Proteinen, welche das Insulin-A-Kette(14-21)-Octapeptid enthalten.

11. Antikörper nach einem oder mehreren der Ansprüche 6 bis 10, dadurch gekennzeichnet, daß sie kreuzreaktiv sind, mit Proteinen, welche das Insulin-A-Kette(16-21)-Hexapeptid enthalten.

12. Antikörper nach einem oder mehreren der Ansprüche 1 bis 11, dadurch gekennzeichnet, daß die Antikörper polyklonal sind.

13. Antikörper nach einem oder mehreren der Ansprüche 1 bis 11, dadurch gekennzeichnet, daß die Antikörper monoklonal sind.

14. Verfahren zur Herstellung der Antikörper nach einem oder mehreren der Ansprüche 1 bis 12, dadurch gekennzeichnet, daß eine geeignete Tierspezies mit einem Peptidbruchstück, welches eine hoch konservierte Aminosäuresequenz eines nativen Proteins darstellt, immunisiert wird und daß man anschließend die Antikörper aus dem Serum der Tierspezies isoliert.

15. Verfahren zur Herstellung der Antikörper nach Anspruch 13, dadurch gekennzeichnet, daß eine geeignete Tierspezies mit einem Peptidbruchstück, welches eine hochkonservierte Aminosäuresequenz eines nativen Proteins darstellt, immunisiert wird, daß man anschließend die Milzzellen dieser immunisierten Tierspezies mit NS1 Myelomzellen fusioniert und die so erzeugten Hybridome auf Sezernierung von monoklonalen Antikörpern gegen das zur Immunisierung eingesetzte Peptidbruchstück selektioniert.

16. Verfahren nach Anspruch 14 oder 15, dadurch gekennzeichnet, daß das zur Immunisierung verwendete Peptidbruchstück an einen Carrier gekoppelt ist.

17. Verwendung der Antikörper nach einem oder mehreren der Ansprüche 1 bis 13 zur Herstellung eines Immunoassays.

18. Immunoassay, dadurch gekennzeichnet, daß er einen oder mehrere Antikörper nach einem oder mehreren der Ansprüche 1 bis 13 enthält.

19. Immunoassay nach Anspruch 18, dadurch gekennzeichnet, daß er ein markiertes Antigen enthält, welches mit dem oder den Antikörpern einen Immunkomplex bildet.

20. Immunoassay nach Anspruch 18, dadurch gekennzeichnet, daß mindestens ein Antikörper markiert ist.

21. Immunoassay nach Anspruch 19 oder 20, dadurch gekennzeichnet, daß die Markierung mit einem radioaktiven, einem chemilumineszenten oder einem enzymatischen Label erfolgt.

22. Immunoassay nach einem oder mehreren der Ansprüche 18 bis 21, dadurch gekennzeichnet, daß ein Antikörper auf einer Festphase immobilisiert ist.

23. Immunoassay nach einem oder mehreren der Ansprüche 18 bis 22, dadurch gekennzeichnet, daß er ein oder mehrere Detergenzien und/oder ein oder mehrere Hilfsproteine und/oder ein oder mehrere Detergens-Hilfsprotein-Mischungen enthält.

24. Immunoassay nach Anspruch 23, dadurch gekennzeichnet, daß er mindestens ein ionisches und mindestens ein nicht-ionisches Detergens enthält.

25. Immunoassay nach einem oder mehreren der Ansprüche 18 bis 24, dadurch gekennzeichnet, daß er außerdem einen hochspezifischen Insulin-Antikörper enthält.

## Patentansprüche (Patentansprüche für folgende(n) Vertragsstaat(en): ES, GR)

1. Verfahren zur Herstellung von Antikörpern, welche durch Immunisierung mit einem Peptidbruchstück, welches eine hochkonservierte Aminosäuresequenz eines nativen Proteins darstellt, dadurch gekennzeichnet, daß eine geeignete Tierspezies mit einem Peptidbruchstück, welches eine hochkonservierte Aminosäuresequenz des nativen Proteins Insulin darstellt, immunisiert wird und daß man anschließend die Antikörper aus dem Serum der Tierspezies isoliert.

2. Verfahren nach Anspruch 1, dadurch gekennzeichnet, daß die Aminosäuresequenz sich an der Oberfläche des nativen Proteins befindet.

3. Verfahren nach Anspruch 1 oder 2, dadurch gekennzeichnet, daß die Aminosäuresequenz geladene und/oder stark polare funktionelle Gruppen enthält.

4. Verfahren nach einem oder mehreren der Ansprüche 1 bis 3, dadurch gekennzeichnet, daß die Aminosäuresequenz mindestens eine Aminosäure enthält, ausgewählt aus: Asn, Asp, Pro, Gly, Gln, Glu.

5. Verfahren nach einem oder mehreren der Ansprüche 1 bis 4, dadurch gekennzeichnet, daß die Aminosäuresequenz 6 - 13 Aminosäuren lang ist.

6. Verfahren nach Anspruch 5, dadurch gekennzeichnet, daß die Aminosäuregruppe 8 Aminosäuren nicht überschreitet und nicht kürzer als 6 Aminosäuren ist.

7. Verfahren nach einem oder mehreren der Ansprüche 1 bis 6, dadurch gekennzeichnet, daß die Aminosäuresequenz in der Insulin-A- oder -B-Kette liegt.

8. Verfahren nach einem oder mehreren der Ansprüche 1 bis 7, dadurch gekennzeichnet, daß das Peptidbruchstück das Insulin-A--A-Kette(14-21)-Octapeptid ist.

9. Verfahren nach einem oder mehreren der Ansprüche 1 bis 8, dadurch gekennzeichnet, daß sie kreuzreaktiv sind mit Proteinen, welche Aminosäuresequenzen enthalten, die mindestens zu 60 - 80 % mit der Aminosäuresequenz des zur Immunisierung eingesetzten Peptidbruchstücks übereinstimmen.

10. Verfahren nach einem oder mehreren der Ansprüche 6 bis 9, dadurch gekennzeichnet, daß sie kreuzreaktiv sind mit Proteinen, welche das Insulin-A-Kette(14-21)-Octapeptid enthalten.

11. Verfahren nach einem oder mehreren der Ansprüche 6 bis 10, dadurch gekennzeichnet, daß sie kreuzreaktiv sind, mit Proteinen, welche das Insulin-A-Kette(16-21)-Hexapeptid enthalten.

12. Verfahren nach einem oder mehreren der Ansprüche 1 bis 11, dadurch gekennzeichnet, daß die Antikörper polyklonal sind.

13. Verfahren nach einem oder mehreren der Ansprüche 1 bis 12, dadurch gekennzeichnet, daß man nach Immunisierung der geeigneten Tierspezies die Milzzellen dieser immunisierten Tierspezies mit NS1 Myelomzellen fusioniert und die so erzeugten Hybridome auf Sezernierung von monoklonalen Antikörpern gegen das zur Immunisierung eingesetzt Peptidbruchstück selektioniert und das die so gewonnenen Antikörper monoklonal sind.

14. Verfahren nach einem oder mehreren der Ansprüche 1 bis 13, dadurch gekennzeichnet, daß das zur Immunisierung verwendete Peptidbruchstück an einen Carrier gekoppelt ist.

15. Verwendung der Antikörper nach einem oder mehreren der Ansprüche 1 bis 14 zur Herstellung eines Immunoassays.

16. Immunoassay, dadurch gekennzeichnet, daß er einen oder mehrere Antikörper nach einem oder mehreren der Ansprüche 1 bis 14 enthält.

17. Immunoassay nach Anspruch 16, dadurch gekennzeichnet, daß er ein markiertes Antigen enthält, welches mit dem oder den Antikörpern einen Immunkomplex bildet.

18. Immunoassay nach Anspruch 16, dadurch gekennzeichnet, daß mindestens ein Antikörper markiert ist.

19. Immunoassay nach Anspruch 17 oder 18, dadurch gekennzeichnet, daß die Markierung mit einem radioaktiven, einem chemilumineszenten oder einem enzymatischen Label erfolgt.

20. Immunoassay nach einem oder mehreren der Ansprüche 16 bis 19, dadurch gekennzeichnet, daß ein Antikörper auf einer Festphase immobilisiert ist.

21. Immunoassay nach einem oder mehreren der Ansprüche 16 bis 20, dadurch gekennzeichnet, daß er ein oder mehrere Detergenzien und/oder ein oder mehrere Hilfsproteine und/oder ein oder mehrere Detergens-Hilfsprotein-Mischungen enthält.

22. Immunoassay nach Anspruch 21, dadurch gekennzeichnet, daß er mindestens ein ionisches und mindestens ein nicht-ionisches Detergens enthält.

23. Immunoassay nach einem oder mehreren der Ansprüche 16 bis 22, dadurch gekennzeichnet, daß er außerdem einen hochspezifischen Insulin-Antikörper enthält.

## Claims (Claims for the following Contracting State(s): AT, BE, CH, DE, DK, FR, GB, IT, LI, LU, NL, SE)

1. An antibody which is obtained by immunization with a peptide fragment which represents a highly conserved amino acid sequence of the native insulin protein.

2. An antibody as claimed in claim 1, wherein the amino acid sequence is located on the surface of the native protein.

3. An antibody as claimed in claim 1 or 2, wherein the amino acid sequence contains charged and/or strongly polar functional groups.

4. An antibody as claimed in one or more of claims 1 to 3, wherein the amino acid sequence contains at least one amino acid selected from: Asn, Asp, Pro, Gly, Gln, Glu.

5. An antibody as claimed in one or more of claims 1 to 4, wherein the amino acid sequence has a length of 6-13 amino acids.

6. An antibody as claimed in claim 5, wherein the amino acid sequence does not exceed 8 amino acids and is not shorter than 6 amino acids.

7. An antibody as claimed in one or more of claims 1 to 6, wherein the amino acid sequence is located in the insulin A or B chain.

8. An antibody as claimed in one or more of claims 1 to 7, wherein the peptide fragment is the insulin A chain (14-21) octapeptide.

9. An antibody as claimed in one or more of claims 1 to 8, which is cross-reactive with proteins which contain amino acid sequences which are at least 60-80 % coincident with the amino acid sequence of the peptide fragment used for the immunization.

10. An antibody as claimed in one or more of claims 6 to 9, which is cross-reactive with proteins which contain the insulin A chain (14-21) octapeptide.

11. An antibody as claimed in one or more of claims 6 to 10, which is cross-reactive with proteins which contain the insulin A chain (16-21) hexapeptide.

12. An antibody as claimed in one or more of claims 1 to 11, which is a polyclonal antibody.

13. An antibody as claimed in one or more of claims 1 to 11, which is a monoclonal antibody.

14. A process for the preparation of the antibodies as claimed in one or more of claims 1 to 12, which comprises immunizing a suitable animal species with a peptide fragment which represents a highly conserved amino acid sequence of a native protein, and subsequently isolating the antibodies from the serum of the animal species.

15. A process for the preparation of the antibodies as claimed in claim 13, which comprises immunizing a suitable animal species with a peptide fragment which represents a highly conserved amino acid sequence of a native protein, subsequently fusing the spleen cells of this immunized animal species to NS1 myeloma cells and selecting the hybridomas thus generated for secretion of monoclonal antibodies against the peptide fragment used for the immunization.

16. The process as claimed in claim 14 or 15, wherein the peptide fragment used for the immunization is coupled to a carrier.

17. The use of the antibodies as claimed in one or more of claims 1 to 13 for the preparation of an immunoassay.

18. An immunoassay which contains one or more antibodies as claimed in one or more of claims 1 to 13.

19. The immunoassay as claimed in claim 18, which contains a labeled antigen which forms an immune complex with the antibody or antibodies.

20. The immunoassay as claimed in claim 18, wherein at least one antibody is labeled.

21. The immunoassay as claimed in claim 19 or 20, wherein labeling is carried out with a radioactive, a chemiluminescent or an enzymatic label.

22. The immunoassay as claimed in one or more of claims 18 to 21, wherein one antibody is immobilized on a solid phase.

23. The immunoassay as claimed in one or more of claims 18 to 22, which contains one or more detergents and/or one or more auxiliary proteins and/or one or more detergent/auxiliary protein mixtures.

24. The immunoassay as claimed in claim 23, which contains at least one ionic and at least one nonionic detergent.

25. The immunoassay as claimed in one or more of claims 18 to 24, which additionally contains one highly specific insulin antibody.

## Claims (Claims for the following Contracting State(s): ES, GR)

1. A process for the preparation of antibodies which are obtained by immunization with a peptide fragment which represents a highly conserved amino acid sequence of a native protein, which comprises immunizing a suitable animal species with a peptide fragment which represents a highly conserved amino acid sequence of the native insulin protein, and subsequently isolating the antibodies from the serum of the animal species.

2. The process as claimed in claim 1, wherein the amino acid sequence is located on the surface of the native protein.

3. The process as claimed in claim 1 or 2, wherein the amino acid sequence contains charged and/or strongly polar functional groups.

4. The process as claimed in one or more of claims 1 to 3, wherein the amino acid sequence contains at least one amino acid selected from: Asn, Asp, Pro, Gly, Gln, Glu.

5. The process as claimed in one or more of claims 1 to 4, wherein the amino acid sequence has a length of 6-13 amino acids.

6. The process as claimed in claim 5, wherein the amino acid group does not exceed 8 amino acids and is not shorter than 6 amino acids.

7. The process as claimed in one or more of claims 1 to 6, wherein the amino acid sequence is located in the insulin A or B chain.

8. The process as claimed in one or more of claims 1 to 7, wherein the peptide fragment is the insulin A chain (14-21) octapeptide.

9. The process as claimed in one or more of claims 1 to 8, wherein the antibodies are cross-reactive with proteins which contain amino acid sequences which are at least 60-80 % coincident with the amino acid sequence of the peptide fragment used for the immunization.

10. The process as claimed in one or more of claims 6 to 9, wherein the antibodies are cross-reactive with proteins which contain the insulin A chain (14-21) octapeptide.

11. The process as claimed in one or more of claims 6 to 10, wherein the antibodies are cross-reactive with proteins which contain the insulin A chain (16-21) hexapeptide.

12. The process as claimed in one or more of claims 1 to 11, wherein the antibodies are polyclonal.

13. The process as claimed in one or more of claims 1 to 12, wherein, after the suitable animal species has been immunized, the spleen cells of this immunized animal species are fused to NS1 myeloma cells and the hybridomas thus generated are selected for secretion of monoclonal antibodies against the peptide fragment used for the immunization, and wherein the antibodies thus obtained are monoclonal.

14. The process as claimed in one or more of claims 1 to 13, wherein the peptide fragment used for the immunization is coupled to a carrier.

15. The use of antibodies as claimed in one or more of claims 1 to 14 for the preparation of an immunoassay.

16. An immunoassay which contains one or more antibodies as claimed in one or more of claims 1 to 14.

17. The immunoassay as claimed in claim 16, which contains a labeled antigen which forms an immune complex with the antibody or antibodies.

18. The immunoassay as claimed in claim 16, wherein at least one antibody is labeled.

19. The immunoassay as claimed in claim 17 or 18, wherein labeling is carried out with a radioactive, a chemiluminescent or an enzymatic label.

20. The immunoassay as claimed in one or more of claims 16 to 19, wherein one antibody is immobilized on a solid phase.

21. The immunoassay as claimed in one or more of claims 16 to 20, which contains one or more detergents and/or one or more auxiliary proteins and/or one or more detergent/auxiliary protein mixtures.

22. The immunoassay as claimed in claim 21, which contains at least one ionic and at least one nonionic detergent.

23. The immunoassay as claimed in one or more of claims 16 to 22, which additionally contains one highly specific insulin antibody.

## Revendications (Revendications pour l'(les) Etat(s) contractant(s) suivant(s): AT, BE, CH, DE, DK, FR, GB, IT, LI, LU, NL, SE)

1. Anticorps caractérisés en ce qu'ils sont obtenus par immunisation avec un fragment peptidique qui représente une séquence d'aminoacides très conservée de la protéine insuline native.

2. Anticorps selon la revendication 1, caractérisés en ce que la séquence d'aminoacides se trouve à la surface de la protéine native.

3. Anticorps selon la revendication 1 ou 2, caractérisés en ce que la séquence d'aminoacides contient des groupes fonctionnels chargés et/ou fortement polaires.

4. Anticorps selon une ou plusieurs des revendications 1 à 3, caractérisés en ce que la séquence d'aminoacides contient au moins un aminoacide choisi parmi: Asn, Asp, Pro, Gly, Gln, Glu.

5. Anticorps selon une ou plusieurs des revendications 1 à 4, caractérisés en ce que la séquence d'aminoacides comporte de 6 à 13 aminoacides.

6. Anticorps selon la revendication 5, caractérisés en ce que la séquence d'aminoacides n'excède pas 8 aminoacides et n'est pas plus courte que 6 aminoacides.

7. Anticorps selon une ou plusieurs des revendications 1 à 6, caractérisés en ce que la séquence d'aminoacides se trouve dans la chaîne A ou B de l'insuline.

8. Anticorps selon une ou plusieurs des revendications 1 à 7, caractérisés en ce que le fragment peptidique est l'octapeptide (14-21) de la chaîne A de l'insuline.

9. Anticorps selon une ou plusieurs des revendications 1 à 8, caractérisés en ce qu'ils présentent une réaction croisée avec des protéines qui contiennent des séquences d'aminoacides coïncidant, à raison d'au moins 60 à 80 %, avec la séquence d'aminoacides du fragment peptidique utilisé pour l'immunisation.

10. Anticorps selon une ou plusieurs des revendications 6 à 9, caractérisés en ce qu'ils présentent une réaction croisée avec des protéines qui contiennent l'octapeptide (14-21) de la chaîne A de l'insuline.

11. Anticorps selon une ou plusieurs des revendications 6 à 10, caractérisés en ce qu'ils présentent une réaction croisée avec des protéines qui contiennent l'hexapeptide (16-21) de la chaîne A de l'insuline.

12. Anticorps selon une ou plusieurs des revendications 1 à 11, caractérisés en ce qu'ils sont polyclonaux.

13. Anticorps selon une ou plusieurs des revendications 1 à 11, caractérisés en ce qu'ils sont monoclonaux.

14. Procédé de production d'anticorps selon une ou plusieurs des revendications 1 à 12, caractérisé en ce que l'on immunise une espèce animale appropriée à l'aide d'un fragment peptidique qui représente une séquence d'aminoacides très conservée d'une protéine native, et on isole ensuite les anticorps à partir du sérum de l'espèce animale.

15. Procédé de production d'anticorps selon la revendication 13, caractérisé en ce que l'on immunise une espèce animale appropriée à l'aide d'un fragment peptidique qui représente une séquence d'aminoacides très conservée d'une protéine native, on fait ensuite fusionner les cellules spléniques de cette espèce animale immunisée avec des cellules de myélome NS1, et on sélectionne les hybridomes ainsi produits sur la base de la sécrétion d'anticorps monoclonaux dirigés contre le fragment peptidique utilisé pour l'immunisation.

16. Procédé selon la revendication 14 ou 15, caractérisé en ce que le fragment peptidique utilisé pour l'immunisation est couplé à une substance porteuse.

17. Utilisation des anticorps selon une ou plusieurs des revendications 1 à 13, pour la préparation d'un dosage immunologique.

18. Dosage immunologique, caractérisé en ce qu'il comporte un ou plusieurs anticorps selon une ou plusieurs des revendications 1 à 13.

19. Dosage immunologique selon la revendication 18, caractérisé en ce qu'il comporte un antigène marqué qui forme avec le ou les anticorps un complexe immun.

20. Dosage immunologique selon la revendication 18, caractérisé en ce qu'au moins un anticorps est marqué.

21. Dosage immunologique selon la revendication 19 ou 20, caractérisé en ce que le marquage est effectué avec un marqueur radioactif, chimiluminescent ou enzymatique.

22. Dosage immunologique selon une ou plusieurs des revendications 18 à 21, caractérisé en ce qu'un anticorps est immobilisé sur une phase solide.

23. Dosage immunologique selon une ou plusieurs des revendications 18 à 22, caractérisé en ce qu'il comporte un ou plusieurs détergents et/ou une ou plusieurs protéines auxiliaires et/ou un ou plusieurs mélanges détergent/protéine auxiliaire.

24. Dosage immunologique selon la revendication 23, caractérisé en ce qu'il utilise au moins un détergent ionique et au moins un détergent non ionique.

25. Dosage immunologique selon une ou plusieurs des revendications 18 à 24, caractérisé en ce qu'il comporte en outre un anticorps d'insuline hautement spécifique.

## Revendications (Revendications pour l'(les) Etat(s) contractant(s) suivant(s): ES, GR)

1. Procédé de production d'anticorps qui sont obtenus par immunisation avec un fragment peptidique représentant une séquence d'aminoacides très conservée d'une protéine native, caractérisé en ce que l'on immunise une espèce animale appropriée à l'aide d'un fragment peptidique qui représente une séquence d'aminoacides très conservée de la protéine insuline native, et en ce que l'on isole ensuite l'anticorps à partir du sérum de l'espèce animale.

2. Procédé selon la revendication 1, caractérisé en ce que la séquence d'aminoacides se trouve à la surface de la protéine native.

3. Procédé selon la revendication 1 ou 2, caractérisé en ce que la séquence d'aminoacides contient des groupes fonctionnels chargés et/ou fortement polaires.

4. Procédé selon une ou plusieurs des revendications 1 à 3, caractérisé en ce que la séquence d'aminoacides contient au moins un aminoacide choisi parmi: Asn, Asp, Pro, Gly, Gln, Glu.

5. Procédé selon une ou plusieurs des revendications 1 à 4, caractérisé en ce que la séquence d'aminoacides comporte de 6 à 13 aminoacides.

6. Procédé selon la revendication 5, caractérisé en ce que la séquence d'aminoacides n'excède pas 8 aminoides et n'est pas plus courte que 6 aminoacides.

7. Procédé selon une ou plusieurs des revendications 1 à 6, caractérisé en ce que la séquence d'aminoacides se trouve dans la chaîne A ou B de l'insuline.

8. Procédé selon une ou plusieurs des revendications 1 à 7, caractérisé en ce que le fragment peptidique est l'octapeptide (14-21) de la chaîne A de l'insuline.

9. Procédé selon une ou plusieurs des revendications 1 à 8, caractérisé en ce que les anticorps présentent une réaction croisée avec des protéines qui contiennent des séquences d'aminoacides coïncidant, à raison d'au moins 60 à 80 %, avec la séquence d'aminoacides du fragment peptidique utilisé pour l'immunisation.

10. Procédé selon une ou plusieurs des revendications 6 à 9, caractérisé en ce que les anticorps présentent une réaction croisée avec des protéines qui contiennent l'octapeptide (14-21) de la chaîne A de l'insuline.

11. Procédé selon une ou plusieurs des revendications 6 à 10, caractérisé en ce que les anticorps présentent une réaction croisée avec des protéines qui contiennent l'hexapeptide (16-21) de la chaîne A de l'insuline.

12. Procédé selon une ou plusieurs des revendications 1 à 11, caractérisé en ce que les anticorps sont polyclonaux.

13. Procédé selon une ou plusieurs des revendications 1 à 11, caractérisé en ce que, après immunisation de l'espèce animale appropriée, on fait fusionner les cellules spléniques de cette espèce animale immunisée avec des cellules de myélome NS1 et on sélectionne les hybridomes ainsi produits, sur la base de la sécrétion d'anticorps monoclonaux dirigés contre le fragment peptidique utilisé pour l'immunisation, et en ce que les anticorps ainsi obtenus sont monoclonaux.

14. Procédé selon une ou plusieurs des revendications 1 ou 13, caractérisé en ce que le fragment peptidique utilisé pour l'immunisation est couplé à une substance porteuse.

15. Utilisation des anticorps selon une ou plusieurs des revendications 1 à 14, pour la préparation d'un dosage immunologique.

16. Dosage immunologique, caractérisé en ce qu'il comporte un ou plusieurs anticorps selon une ou plusieurs des revendications 1 à 14.

17. Dosage immunologique selon la revendication 16, caractérisé en ce qu'il comporte un antigène marqué qui forme avec le ou les anticorps un complexe immun.

18. Dosage immunologique selon la revendication 16, caractérisé en ce qu'au moins un anticorps est marqué.

19. Dosage immunologique selon la revendication 17 ou 18, caractérisé en ce que le marquage est effectué avec un marqueur radioactif, chimiluminescent ou enzymatique.

20. Dosage immunologique selon une ou plusieurs des revendications 16 à 19, caractérisé en ce qu'un anticorps est immobilisé sur une phase solide.

21. Dosage immunologique selon une ou plusieurs des revendications 16 à 20, caractérisé en ce qu'il comporte un ou plusieurs détergents et/ou une ou plusieurs protéines auxiliaires et/ou un ou plusieurs mélanges détergent/protéine auxiliaire.

22. Dosage immunologique selon la revendication 21, caractérisé en ce qu'il comporte au moins un détergent ionique et au moins un détergent non ionique.

23. Dosage immunologique selon une ou plusieurs des revendications 16 à 22, caractérisé en ce qu'il comporte en outre un anticorps d'insuline hautement spécifique.
